# EUROPEAN PATENT SPECIFICATION

(11) **EP 2 544 762 B1**
(45) Date of publication and mention of the grant of the patent: **25.12.2013**
(21) Application number: 11707981.4
(22) Date of filing: 03.03.2011
(51) Int. Cl.: A61N 1/37, A61N 1/39, A61B 5/0452

(54) **INTRACARDIAC ELECTROGRAM TIME FREQUENCY NOISE DETECTION**
DETEKTION VON ZEITFREQUENZRAUSCHEN EINES INTRAKARDIALEN ELEKTROGRAMMS
DÉTECTION DE BRUIT DE FRÉQUENCE TEMPORELLE D'ÉLECTROGRAMME INTRACARDIAQUE

(30) Priority: 09.03.2010 US 312064 P
(43) Date of publication of application: 16.01.2013
(73) Proprietor: Cardiac Pacemakers, Inc., St. Paul, MN 55112-5798 (US)
(72) Inventor: MAHAJAN, Deepa, Circle Pines Minnesota 55014 (US); LIU, Chenguang, Minneapolis Minnesota 55305 (US); DONG, Yanting, Lexington, KY 40513 (US)
(74) Representative: Pfenning, Meinig & Partner GbR
(86) International application number: PCT/US2011/027036
(87) International publication number: WO 2011/112420

(56) References cited:
- WO-A1-2008/137539
- US-A1- 2004 106 957
- US-B1- 6 434 417

## Description

### BACKGROUND

An implantable medical device (IMD) can be used to monitor a physiological parameter or provide therapy, such as to elicit or inhibit a muscle contraction, or to provide neural stimulation, or for therapeutic or diagnostic uses. An example of an IMD can include a cardiac rhythm management (CRM) device, which can be configured to treat disorders of cardiac rhythm. For example, a CRM can help by sensing a patient's heart rhythm, detecting tachycardia or fibrillation, providing pacing electrostimulations to evoke responsive heart contractions, providing cardiac resynchronization therapy (CRT) electrostimulations to coordinate the spatial nature of a heart contraction of one or more heart chambers, providing antitachyarrhythmia pacing, cardioversion, or defibrillation shocks to interrupt a tachyarrhythmia.

### OVERVIEW

The invention is set out in the appended claims. The aspects, examples or embodiments of the present description which do not fall within the scope of said claims are provided for illustrative purposes only and do not form part of the present invention.

In some instances, therapy, such as anti-arrhythmia therapy, can be delivered to a patient when the patient has not experienced any of the symptoms related to the delivered therapy. In these instances, the CRM device may have inaccurately detected an episode that did not occur and may compromise the patient's safety and reduce the patient's quality of life due to unnecessary therapy delivery. This inaccurate detection may be caused by oversensing, or inaccurate detection of events within a cardiac cycle, and may be generally classified into two categories: oversensing based on physiological signals, and oversensing based on non-physiological signals. Physiological signals include myopotentials such as noise created by the surrounding muscle tissue. Non-physiological signals include electromagnetic interference, fractured leads, loose screw sets, poor connectivity of a lead, or failed lead insulation.

Oversensing in a CRM device can be a rare occurrence, and differentiating between an actual arrhythmia and whether oversensing has occurred can be difficult to assess since the device can be properly detecting arrhythmias a majority of the time. In order to mitigate delivery of unnecessary therapy to the patient due to oversensing, the CRM device can apply a classification scheme to determine occurrences of oversensing and identify and classify specific forms of oversensing.

An intracardiac electrogram (IEGM) can record changes in electrical potentials of a specific location of the heart as measured with electrodes of a CRM device placed in, on, and/or near the heart. In an example which does not form part of the present invention, an offline classification method, such as for classifying noise signals collected from an electrogram can be used to provide a programming recommendation for the CRM device. The classification method can determine a classification based on morphology information in a time domain, frequency components in a frequency domain, and energy distribution in a time-frequency domain. An intrinsic cardiac signal can have distinct energy distribution characteristics in the time-frequency domain when compared to the energy distribution of a noise signal in certain channels. This information can be used by a physician or trained professional for better management of the IMD (e.g., detecting and classifying the noise as due to a malfunctioning lead).

Example 1 describes subject matter that can use or include a system comprising a first identification circuit configured to identify a first noise characteristic associated with a sensing channel signal and a noise classification circuit configured to determine a noise classification using the first noise characteristic and using information about energy distribution in a time-frequency domain of the sensing channel signal.

In Example 2, the subject matter of Example 1 can optionally include a second identification circuit configured to identify a second noise characteristic, associated with a ventricular channel signal different from the sensing channel signal, using the first noise characteristic, wherein the noise classification circuit is configured to determine a noise classification using the first and second noise characteristics and using information about energy distribution in the time-frequency domain of at least one of the sensing channel signal or the ventricular channel signal.

In Example 3, the subject matter of Examples 1 or 2, can optionally include a third identification circuit configured to identify a third noise characteristic, associated with a atrial channel signal, using the first and second noise characteristics; wherein the noise classification circuit is configured to determine noise classification using the first, second, and third noise characteristics and using information about the energy distribution in a time-frequency domain of at least one of the sensing channel signal, the ventricular channel signal, or the atrial channel signal.

In Example 4, the subject matter of any one of Examples 1-3 can optionally include a device programming circuit configured to provide a device programming or recommendation using the determined noise classification.

In Example 5, the subject matter of any one of Example 1-4 can optionally include the first identification circuit being configured to analyze the sensing channel signal for noise based on comparing a heart rate to an arrhythmia range.

In Example 6, the subject matter of any one of Examples 1-5 can optionally include the first identification circuit being configured to analyze the sensing channel signal for noise by applying a filter to the sensing channel signal and determining the energy distribution above a specified frequency.

In Example 7, the subject matter of any one of Examples 1-6 can optionally include the second identification circuit being configured to analyze the ventricular channel signal for noise in a first interval when a heart rate is lower than or equal to a threshold to determine noise between cardiac depolarizations, and to analyze data in a second interval when the heart rate is higher than the threshold to determine noise within cardiac cycles.

In Example 8, the subject matter of any one of Examples 1-7 can optionally include the second identification circuit configured to analyze the ventricular channel signal for noise in the first interval to further including determining whether there is noise in the interval by comparing an amplitude of the ventricular channel signal to a first threshold.

In Example 9, the subject matter of any one of Example 1-8 can optionally include wherein the second identification circuit is configured to analyze the ventricular channel signal for noise in the second interval including applying a time-frequency transform to the ventricular channel signal, and determining a time instant having a maximal energy level and deriving a variability indicator of the time instant and comparing the variability indicator to a second threshold.

In Example 10, the subject matter of any one of Examples 1-9 can optionally include a method comprising identifying a first noise characteristic associated with a sensing channel signal using a circuit, determining a noise classification using the first characteristic and using information about an energy distribution in a time-frequency domain of the sensing channel signal.

In Example 11, the subject matter of any one of Examples 1-10 can optionally include identifying a second noise characteristic, associated with a ventricular channel signal different from the sensing channel signal, using the first noise characteristic, and determining a noise classification using the first and second noise characteristics and using information about energy distribution in the time-frequency domain of at least one of the sensing channel signal or the ventricular channel signal.

In Example 12, the subject matter of any one of Examples 1-11 can optionally include identifying a third noise characteristic, associated with a atrial channel signal, using the first and second noise characteristics; wherein the noise classification circuit is configured to determine noise classification using the first, second, and third noise characteristics and using information about the energy distribution in a time-frequency domain of at least one of the sensing channel signal, the ventricular channel signal, or the atrial channel signal.

In Example 13, the subject matter of any one of Examples 1-12 can optionally include providing a device programming or recommendation using the determined noise classification.

In Example 14 the subject matter of any one of Examples 1-13 can optionally include analyzing the ventricular channel signal for noise in a first interval when a heart rate is lower than or equal to a threshold to determine noise between cardiac depolarizations and analyzing data in a second interval when the heart rate is higher than the threshold to determine noise within a cardiac cycle.

In Example 15, the subject matter of any one of Examples 1-14 can optionally include analyzing the ventricular channel signal for noise in the first interval to further include determining whether there is noise in the interval by comparing an amplitude of the ventricular channel signal to a first threshold.

In Example 16, the subject matter of any one of Examples 1-15 can optionally include analyzing the ventricular channel signal for noise in the second interval to further include applying a time-frequency transform to the ventricular channel signal, and includes determining a time instant having a maximal energy level and deriving a variability indicator of the time instant and comparing the variability indicator to a second threshold.

In Example 17, the subject matter of any one of Examples 1-16 can optionally include an apparatus comprising a first identification circuit configured for identifying a first noise characteristic associated with a shock channel signal; a second identification circuit configured for identifying a second noise characteristic associated with a ventricular rate sensing channel signal, using the first noise characteristic; a third identification circuit configured for identifying a third noise characteristic, associated with a atrial rate sensing channel signal, using the first and second noise characteristics; and a noise classification circuit for determining a noise classification using the first, second, and third noise characteristics using information about an energy distribution in a time-frequency domain of at least one of the shock channel signal, the ventricular rate sensing channel signal, or the atrial rate sensing channel signal.

In Example 18, the subject matter of any one of Examples 1-17 can optionally include the second identification circuit is configured to analyze the ventricular channel signal for noise in a first interval when a heart rate is lower than or equal to a threshold to determine noise between cardiac depolarizations, and to analyze the ventricular channel signal for noise in a second interval when the heart rate is higher than the threshold to determine noise within a cardiac cycle.

In Example 19, the subject matter of any one of Examples 1-18 can optionally include the second identification circuit is further configured to apply a time-frequency transform to the ventricular signal, and determine a time instant having a maximal energy level.

In Example 20, the subject matter of any one of Examples 1-19 can optionally include the second identification circuit is configured to analyze the ventricular channel signal for noise in the second interval by determining whether a frequency distribution in the shock channel signal is within a narrow band and analyzing an energy distribution of the shock channel signal by applying a time frequency transform to the signal.

WO 2008/137539 describes a system comprising a noise classification circuit using multiple sequential noise tests. US-6 434 417 relates to noise detection using the energy distribution of a time-frequency transform of a sensed signal.

### BRIEF DESCRIPTION OF THE DRAWINGS

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.
FIG. 1 shows an example of an intra cardiac electrogram output displaying oversensing based on a non-physiological external noise in two channels;
FIGS. 2A-2F illustrate shock channel signals and corresponding energy distributions of the signal;
FIGS. 3A-3F illustrate ventricular sensing channel signals and corresponding energy distributions of the signals;
FIG. 4 illustrates an example of signal classification using three channels;
FIG. 5 illustrates an example of determining noise in the shock channel such as by analyzing the density of significant points of the shock channel signal over a period of time and by time-frequency analysis;
FIG. 6 illustrates an example of determining noise in the ventricular channel;
FIG. 7 is an example of determining noise in the ventricular channel;
FIG. 8 illustrates an example of determining noise in the atrial channel;
FIG. 9 illustrates an example of an implantable medical device including an intracardiac electrogram module for classifying noise;
FIG. 10 is a block diagram illustrating an example of an implantable cardiac function management system communicatively coupled to an external communication device;
FIG. 11 is a block diagram illustrating an example of an implantable cardiac function management system configured to enable adaptive data storage and download;
FIG. 12 a block diagram illustrating an example of an external programming and diagnostic computer.

### DETAILED DESCRIPTION

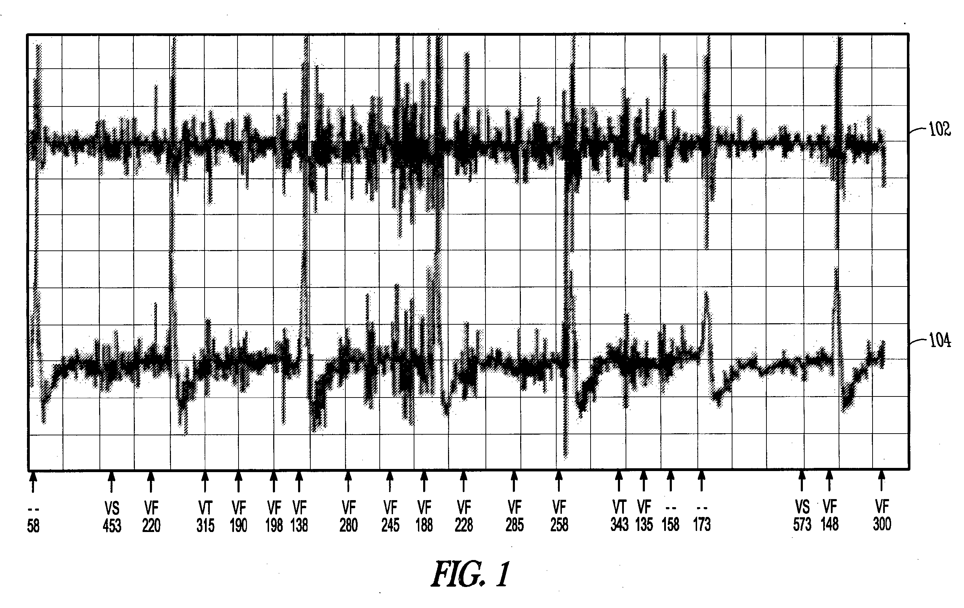

FIG. 1 shows an example of an intra-cardiac electrogram output displaying oversensing based on a non-physiological external noise in two channels. The first channel 102 can be a ventricular rate channel while the second channel 104 can be a ventricular shock channel. The morphologies of the signals in the time domain, and the frequencies and the amplitudes of the signals in the frequency domains of the first channel 102 and the second channel 104 can be used to determine whether these signals are produced by a normal heart rhythm, arrhythmia, or actual noise. As such, FIG. 1 illustrates noise in both the first and second channels 102 and 104. Because there is noise in both channel and based on the frequency and amplitude of the noise in both first and second channels 102 and 104 it can be determined that this noise can be caused by a non-physiological external source, such as, for example, electromagnetic interference. In addition to classification based on the morphology and the frequency components as described above, classification of the signals can be enhanced by using this information and further analyzing the energy distribution in the time-frequency domain. Once noise is detected, this information can be reported to a physician, clinician or provided as data to an algorithm, such as for determining action, such as, for example, withholding tachyarrhythmia therapy, change device programming, changing a parameter of the therapy, etc.

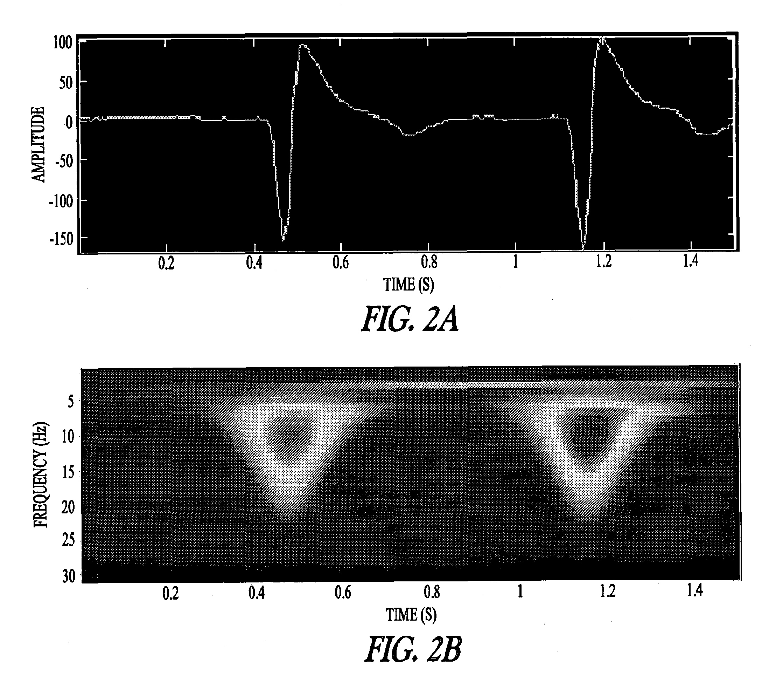

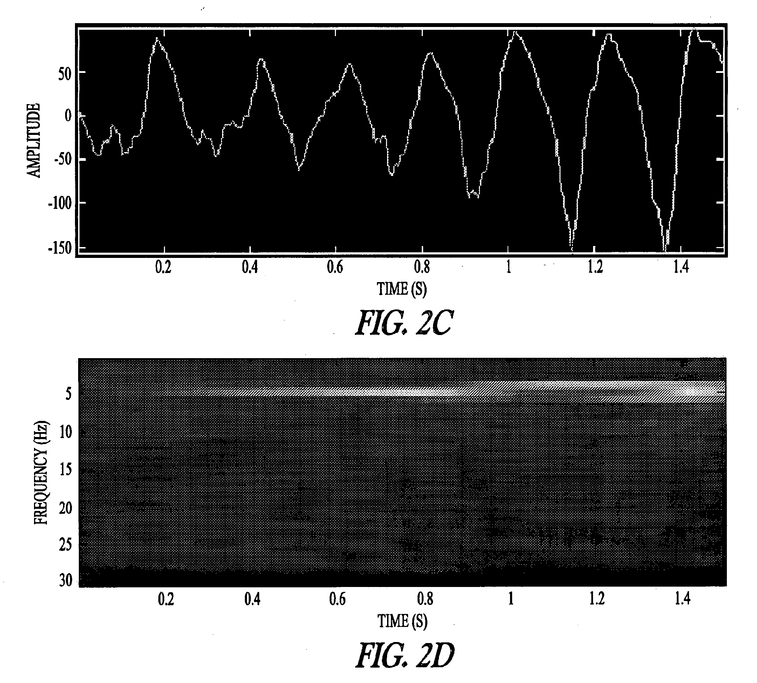

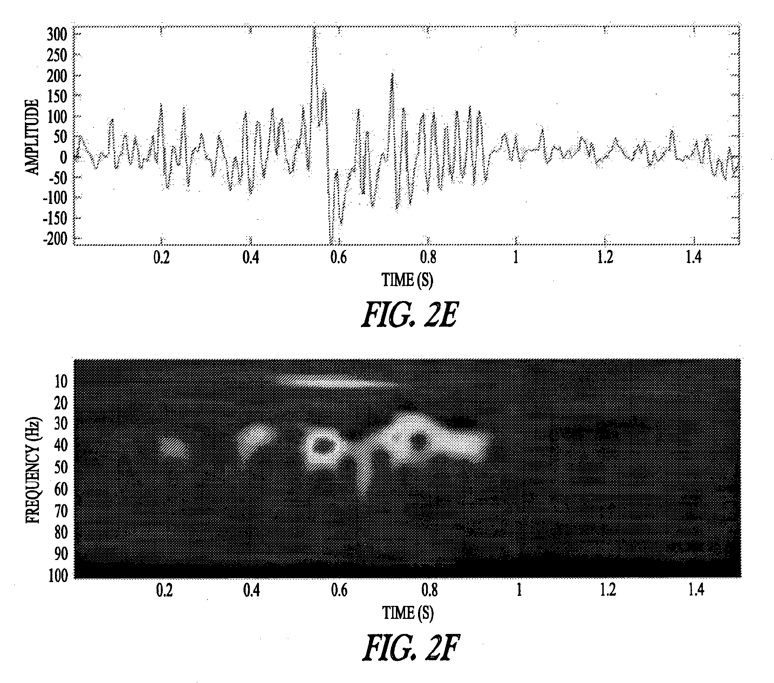

FIGS. 2A-2F illustrate shock channel signals and corresponding energy distributions of the signals. Shock channel, with a bandwidth of 3Hz to 80Hz, is an example of a wide-band, non-bipolar sensing signal. As illustrated, the time is measured in the x-axis of the graph for FIGS. 2A - 2F; the signal amplitude is measured in the y-axis for FIGS. 2A, 2C, and 2E; the frequency is measured in the y-axis for FIGS. 2B, 2D and 2F. FIGS. 2A-2B depict a wave form and the corresponding energy distribution of the wave form of a normal heart signal in the time-frequency domain. Various time-frequency analysis techniques may be used, such as wavelet transform or Wigner transform when determining noise in the signal. FIGS. 2C-2D depict a wave form and the corresponding energy distribution of the wave form of a ventricular tachycardia signal in the time-frequency domain. FIGS. 2E-2F depict a wave form and the corresponding energy distribution of the wave form of a ventricular noise signal in the time-frequency domain. By comparing FIGS. 2A, 2C and 2E, it can be seen that the noise signals have more signal turns (e.g., significant points, directional changes) than normal heart rhythm and ventricular tachycardia signals. This is reflected in FIGS. 2B, 2D, and 2F: the majority of the signal components from normal sinus and ventricular tachycardia can have a frequency range of less than 30Hz, while the main signals components of the noise signals is above 30Hz. In FIG 2D, it can also be seen that the energy of the tachycardia is mainly distributed in a narrow frequency band.

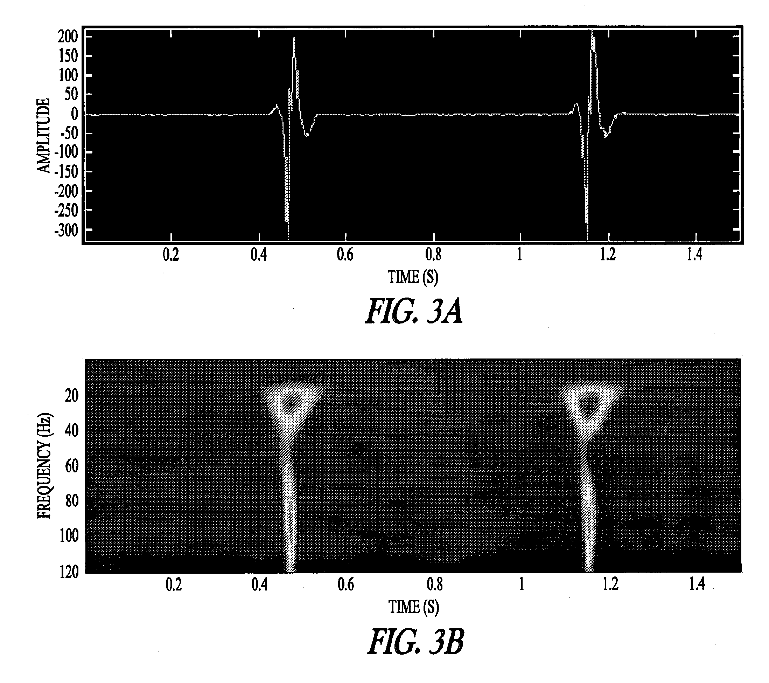

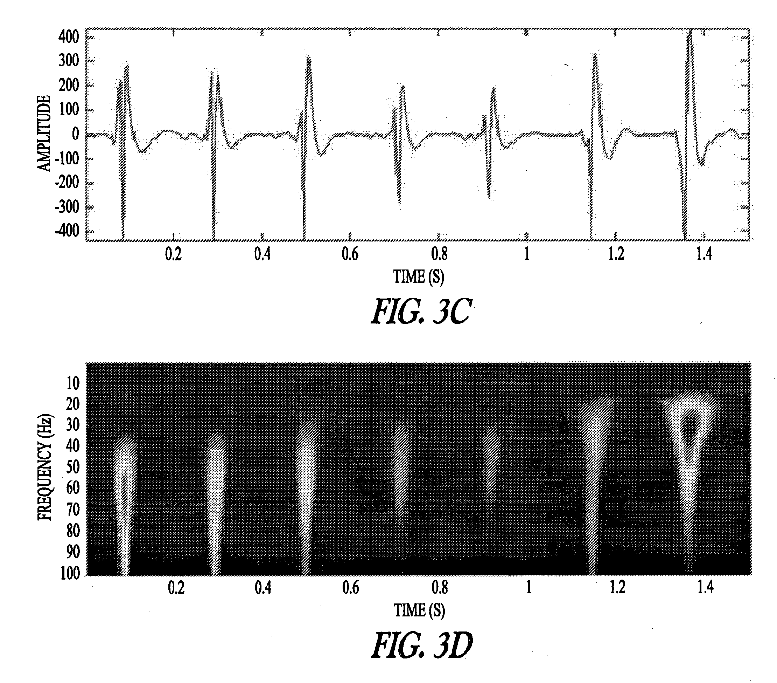

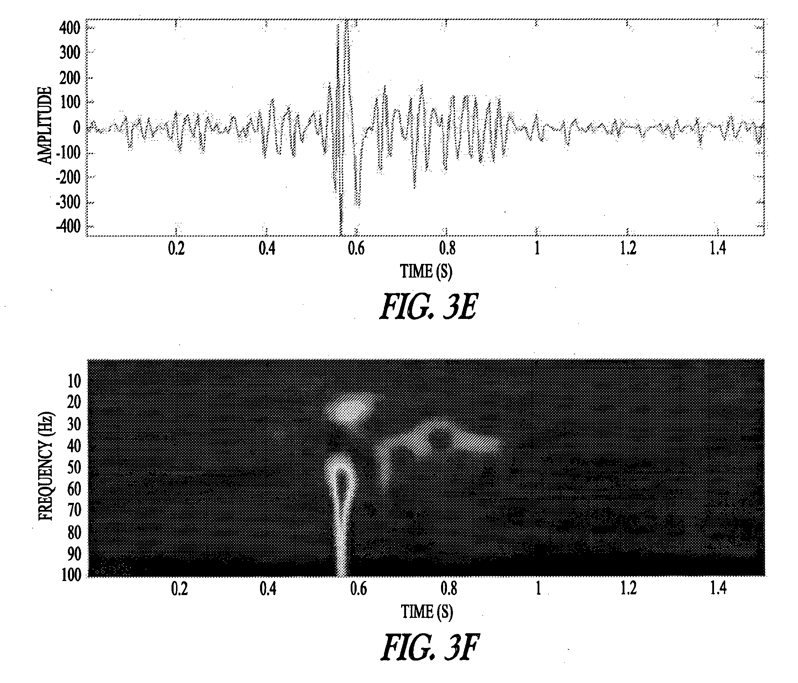

FIGS. 3A-3F illustrate ventricular sensing channel signals and corresponding energy distributions of the signals. The rate sensing channel, with an example bandwidth of 20Hz to 170 Hz, is an example of a narrow-band sensing signal, which can be both bipolar and non-bipolar. As illustrated, the time can be measured in the x-axis of the graph for FIGS 3A-3F; the signal amplitude can be measured in the y-axis for FIGS 3A, 3C, and 3E; the frequency can be measured in the y-axis for FIGS 3B, 3D and 3F. FIGS. 3A-3B depict a wave form and the corresponding energy distribution of the wave form of a normal heart signal in the time-frequency domain. FIGS. 3C-3D depict a wave form and the corresponding energy distribution of the wave form of a ventricular tachycardia signal in the time-frequency domain. FIGS. 3E-3F depict a wave form and the corresponding energy distribution of the wave form of a ventricular noise signal in the time-frequency domain. By comparing FIGS. 3A, 3C and 3E, it can be seen that the noise signals have much more signal turns than normal sinus and ventricular tachycardia signals. More number of turns in a signal corresponds to higher density of significant points (SP). SPs are special points along the signal which best represent the "turns" taken by the signal over time. U.S. Patent 6,950,702 describes determination and application of significant points of a signal. In addition, in the non-noise signals, there is a resting period between the consecutive heart beats, while there is no resting period in the noise signals. Similar observations can be seen in FIGS 3B, 3D, and 3F: the normal sinus and ventricular tachycardia have the majority of the energy concentrated around the heart beat, while the energy distribution of the noise signals are more disperse. Those characteristics can also be observed in the atrial narrow-band sensing signals.
Based on FIGS. 2A-F and 3A-F, time frequency analysis of different channels can be very useful in classifying a signal as either noise or physiological signals.

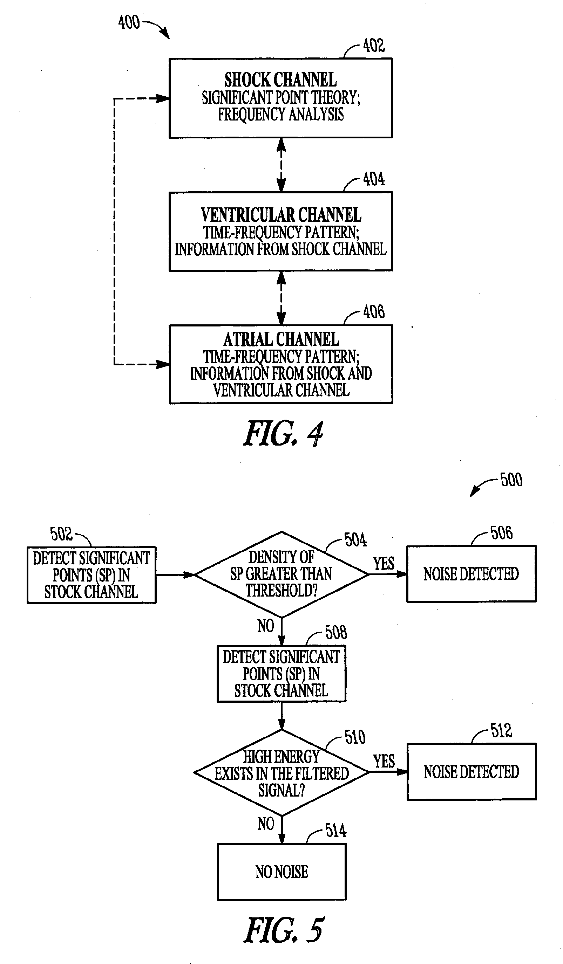

FIG. 4 illustrates an example 400 of signal classification using three channels. At 402, noise in the shock channel can be detected, such as by using significant point method and frequency analysis. At 404, noise in the ventricular channel can be detected, such as by using time frequency patterns and information from shock channel. At 406, noise in the atrial channel can be detected using time-frequency patterns and information from shock and ventricular channel. The information from all three channels can be collectively used to classify a signal. In some examples, any one or combination of the channels can be used to classify a signal as noise.

FIG. 5 illustrates an example 500 of determining noise in the shock channel signal, such as by analyzing the density of significant points of the shock channel signal over a period of time and by time-frequency analysis. In the example, at 502, SP in the shock channel signal can be detected. At 504, it can be determined whether the density of significant points is greater than a threshold for a given time period. If at 504, the density of significant points is greater than the threshold, then at 506, the method can determine that noise is detected in the shock channel. If at 504, the density of significant points is not greater than the threshold, then at 508, the method can apply a high pass filter to the signal. In an example, a signal can be filtered with a 30Hz high pass filter, while in other examples a different filter can be applied to the signal. 30Hz can be used since, the noise signal has frequency distribution of higher than 30Hz, while the physiological signals has a frequency distribution of lower than 30Hz. At 510, it can be determined whether high energy exists in the filtered signal, and if so, at 514, it can be declared that noise is in the shock channel signal. If at 510, it is determined that high energy does not exist in the filtered signal, then it can be declared that no noise is in the shock channel signal.

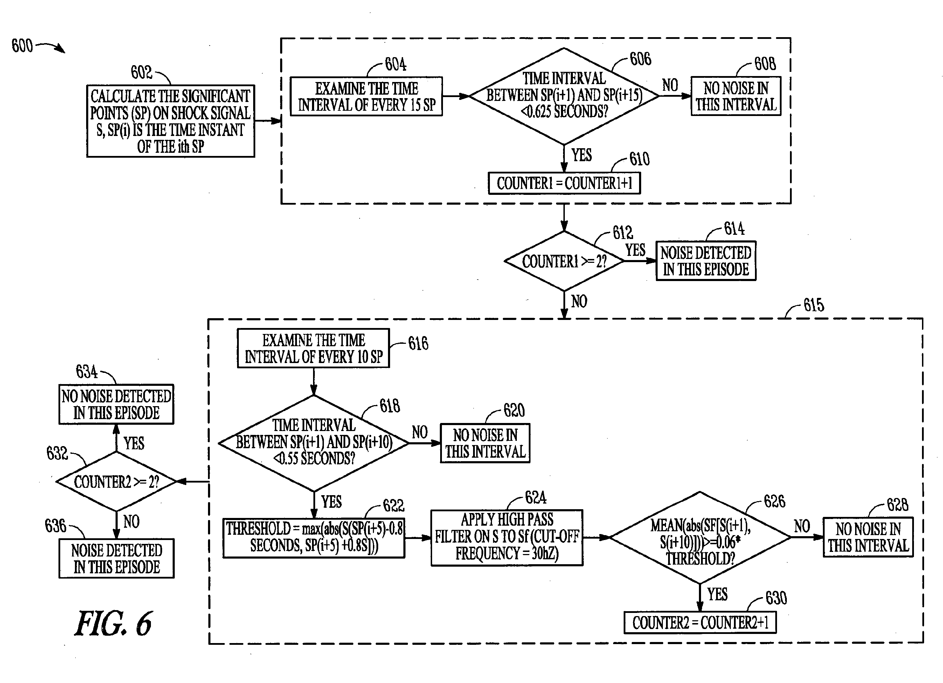

FIG. 6 illustrates an example 600 of determining noise in the shock channel based on significant point analysis and time frequency analysis. In the example, at 602, the method can calculate the significant points of the shock signal over a time interval. For example, at 604, the method can determine a time interval difference between the first and the fifteenth significant point, and at 606, the method can determine whether the time interval is less than 0.0625 seconds. If the time interval between the first and the fifteenth significant points is not less than 0.0625 seconds, then at 608 the method can determine there is no noise in the interval. If the time interval between the first and the fifteenth significant points is less than 0.0625 seconds, then at 610 the method can increment a counter by 1, and the method can determine another interval within the episode and can examine it also. At 612, if the counter is equal to or exceeds two, then at 614 the method can determine that noise is detected in the episode. If, however at 612, the counter is less than two, then the method can perform a more sensitive analysis of the episode at 615.

Within 615, the method can perform a more detailed analysis of the interval and particularly, at 616, the method can examine the time interval of 10 significant points. At 618, the method can determine whether the time interval of the 10 significant points is less than 0.55 seconds. If not, then at 620, the method can determine there is no noise in the interval. If there are 10 significant points in a 0.55 second interval, then at 622, the method can determine a threshold value using a point in time where the fifth significant point is detected. At 624, the method can apply a high pass filter to the signal with an example cut-off frequency of 30Hz. High pass filter passes frequencies higher than the cut-off frequency well but attenuates the frequencies lower than the stop frequency. Cut-off frequency can be chosen based on the implementation of filters setting within an implantable device or any device used for recording the signal. At 626 if the average energy of the filtered signal within a time interval (SF) between the 10 significant points is greater than or equal to the calculated fraction of the threshold, then a second counter is incremented by one. Otherwise, if the average energy of the filtered signal (SF) between the first and 10^{th} significant points is less than the calculated fraction of the threshold, the method can determine that no noise can be in the interval. At 632, when the second counter is greater than or equal to two, the method can determine that noise is detected in the episode at 634. When at 632, the second counter is less than two, the method can determine that noise is not detected in this episode. Essentially after applying a filter, if energy exists in a higher frequency, this can be an indication that there is noise, and if there are two such instances, this can be a greater indicator of noise in the channel. All values used are used by way of example, and various other values can be used in place. For example, the cut off frequency, the significant points time interval can be refined and adjusted as would be obvious to one of ordinary skill for the purposes sought.

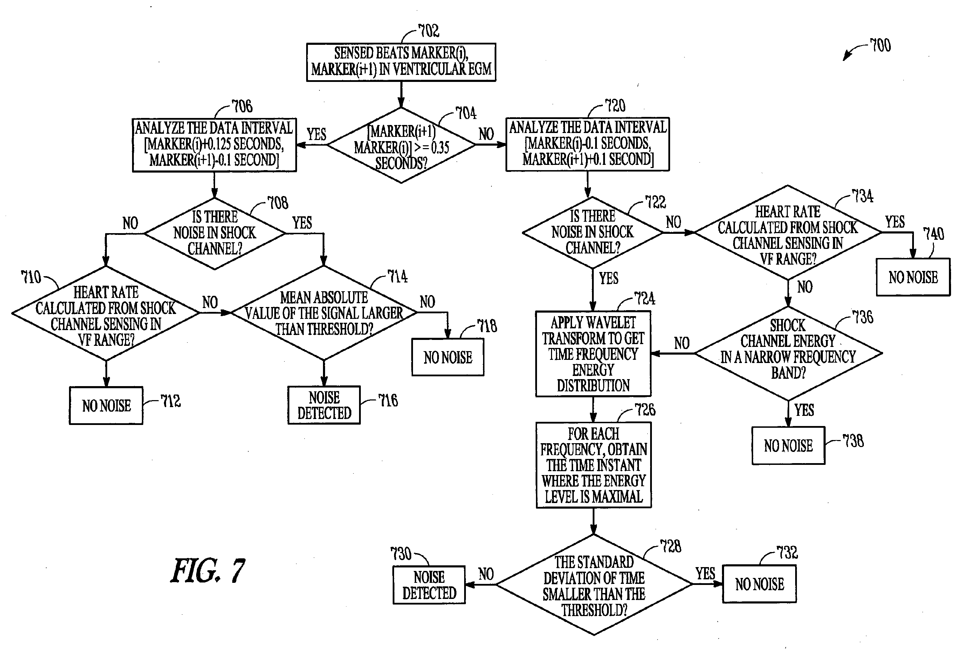

The ventricular channel noise detection method can determine whether there is a potential noise in the channel by looking at the time interval between two consecutive beats. Time between consecutive beats represents a physiological phenomenon of the contracting and relaxing of the ventricles. If time between two consecutive beats is less than a pre-specified threshold then it could be a noise. However, it will still not be reported as noise. Additional criterion is used to make sure it is real noise and not ventricular fibrillation. This additional criterion uses information from a shock channel. If the shock channel is noisy, then the method can determine that there is noise detected in the ventricular channel, since it is very likely that ventricular sensing signals is noisy if the shock channel is also noisy. If, however, the shock channel is not noisy, then the method can determine whether the shock channel has characteristics of ventricular fibrillation. Some of the characteristics of ventricular fibrillation include fast heart rate, energy of the shock channel signal in time frequency domain. If the shock channel has characteristics of ventricular fibrillation, then there can be no noise, and instead, the method can determine there is ventricular fibrillation in the signal. In some embodiments, if the shock channel includes characteristics of ventricular fibrillation, both noise and ventricular fibrillation can be detected. If however, the shock channel does not include characteristics of ventricular fibrillation, the method can determine that there is noise detected in the ventricular channel. The determination of ventricular fibrillation can be based on filtering the signal for the channel in the time frequency domain, and determining whether the channel signal exceeds a threshold frequency as will be discussed with reference to FIG. 7.

FIG. 7 is an example 700 of determining noise in the ventricular channel. At 702, the method can determine the time between consecutive sensed beats in the ventricular electrogram. If at 704, the difference in time between the consecutively sensed beats is greater than or equal to a threshold, in this case, 0.35 seconds, the method can continued to analyze the data interval in greater detail at 706. At 706 the method can analyze a smaller interval within the consecutive beat markers, such as in this case, narrowed by adding 0.125 seconds to the first beat marker and subtracting 0.1 seconds to the second in time of the next beat marker. Most likely, the chosen interval reflects the resting period between the consecutive beats. As in FIG 3, the resting period of the physiological signals can be mostly "quiet", with very small signal amplitude, while there may be no resting period for the noise signals. At 708, if there is no noise in the shock channel, then at 710 the method determines whether the calculated heart rate from shock channel sensing is in the ventricular fibrillation range. If so, there is no noise in the ventricular channel. If at 710, the heart rate calculated from the shock channel sensing is not within the ventricular fibrillation range, then at 714 the method can determine whether the mean absolute value of the signal is larger than a threshold. The method can also determine whether the mean absolute value of the ventricular signal is larger than the threshold when the method has determined that there is noise in the shock channel at 708. If the mean absolute value of the ventricular signal is larger than the threshold, then at 716, the method can determine noise is detected in the ventricular channel. If the mean absolute value of the ventricular signal is not greater than the threshold, then the method can determine that no noise is in the ventricular channel.

Returning to 704, if the method can determine the interval between consecutive sensed beats is less than, the threshold, in this case, 0.35 seconds, the method can continue to 720. At 720, the method can analyze a broader interval by expanding the interval by subtracting 0.1 second to the first marker, and by adding 0.1 seconds to the second marker in time. This interval most likely includes signals around the heart beat. According to FIG 3, the frequency distribution of the physiological signals, such as normal heart rhythm, or ventricular tachycardia, has a frequency distribution that is concentrated around the heart beat, while the frequency distribution of noise signals can be widely distributed and spread out. If at 722, the method has determined that there is noise in the shock channel (see the analysis of shock channel with respect to FIGS. 4 and 6), then at 724, the method can transform the ventricular signal from time domain to time-frequency domain using techniques such as a wavelet transform, to determine the time frequency energy distribution. At 726, the method obtains the time instant where the energy is at the maximum level for each frequency and continues to 728. At 728, if the standard deviation of time instances is not smaller than a threshold, suggesting that the frequency distribution is more spread, then at 730, the method can determine that noise detected in the ventricular channel. If, however, at 728, the standard deviation of time instances is smaller than a threshold, suggesting that the frequency distribution is more concentrated, then at 732, the method can determine that noise is not detected in the ventricular channel.

Returning to 722, if the method can determine no noise is in the shock channel, the method continued to 734, where the method can determine whether the heart rate calculated from the shock channel sensing is within the ventricular fibrillation range. If so, then at 740, the method can determine that no noise is in the ventricular channel. If at 734, the method determines the heart rate calculated from the shock channel is not in the ventricular fibrillation range, then at 736, the method can determine whether the shock channel energy is in a narrow frequency band. Since the shock channel energy is concentrated in a narrow band for the ventricular tachycardia, as shown in FIG. 2C & 2D, therefore, if this is true, there is no noise, but if the shock channel energy is not in a narrow frequency band, then the method can continue to 724 and can apply the transform as described above. In some examples, the detection of noise in the ventricular channel signal can be determined without analyzing the shock channel.

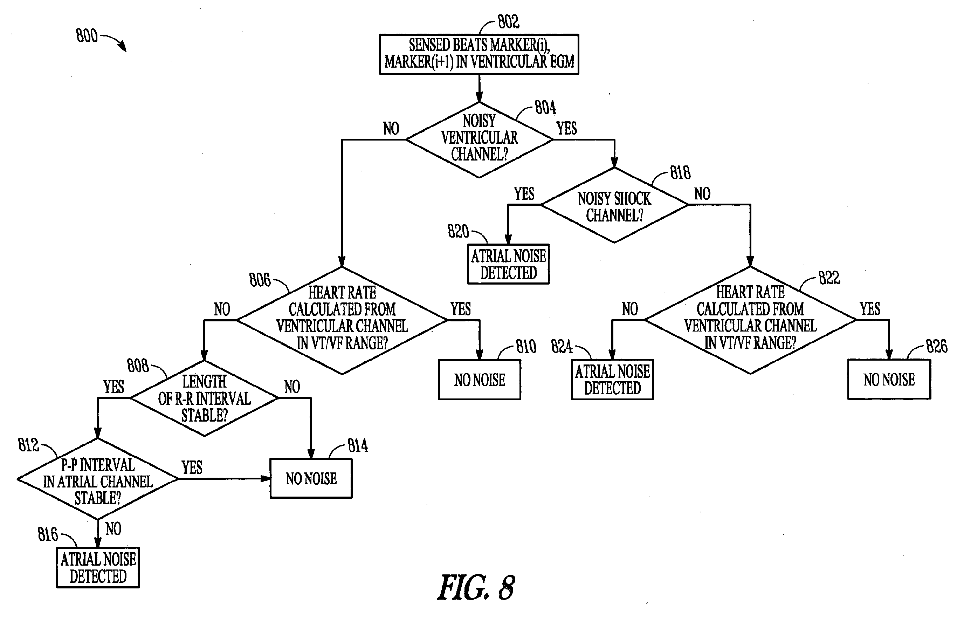

FIG. 8 illustrates an example 800 of determining noise in the atrial channel. When determining noise in the atrial channel, the method combines the methods of determining noise in the shock channel as described above with respect to FIGS. 2A-F and 5, and the ventricular channel as described above with respect to FIG.7. At 802, the method can detect a suspicious segment from the atrial channel using time-frequency analysis on atrial channel. This analysis is similar to the one explained in 724, 726 and 728. At 804, the method can determine whether the ventricular channel was noisy using method described in FIG 7. If at 804, the method determines the ventricular channel is not noisy, then, at 806, the method can determine whether the heart rate calculated from the ventricular channel is within the ventricular tachyarrhythmia/fibrillation range. If so, then at 810 the method can determine there is not noise in the atrial channel. If, however, the heart rate calculated from the ventricular channel is not within the range, the method can continue to 808, and the method can determine whether the length of the R-R interval is stable. If it is not stable, then at 814 the method can determine no noise exists in the atrial channel, since an unstable R-R interval is an indication of atrial fibrillation. If at 808, the method determines that the R-R interval is stable, the method continues to 812 and analyzes the P-P interval of the atrial channel for stability. If at 812, the method can determine the P-P interval is stable, then at 814 the method can conclude no noise is in the atrial channel. However, if the P-P interval is not stable, then at 816, the method can conclude there is atrial noise.

If at 804, there is a noisy ventricular channel, then at 818, the method can determine whether there is a noisy shock channel from the analysis above with respect to FIGS. 2A-F and 5. If, from the analysis, there is a noisy shock channel, then at 820, the method can determine that atrial noise is detected. If however, the analysis of the shock channel is determined to be not noisy, the method continues to 822, and the method can determine whether the heart rate calculated from the shock channel is within the ventricular tachyarrhythmia/fibrillation range. If it is not within the range, then the method can determine there is noise in the atrial channel at 830, since most likely the ventricular rhythm is fast when the atrial rhythm is fast. If the heart rate is within the range, the method can determine there is not noise in the atrial channel. The heart rate range can be acceptably known ranges to those of ordinary skill. In some examples, the range can be set to increase sensitivity of the method. In some examples, atrial channel signal noise can be determined without analyzing the shock channel and without analyzing the ventricular channel.

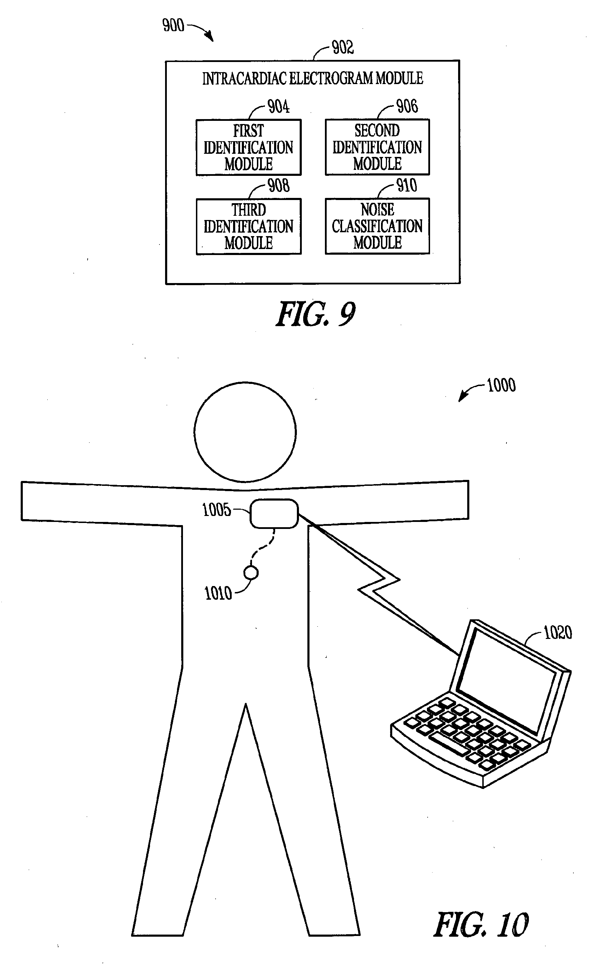

FIG. 9 illustrates an example 900 of an intracardiac electrogram module 902 for classifying noise. In the example, the intracardiac electrogram module can include a first identification module 904, a second identification module 906, a third identification module 908 and a noise classification module 910. The first identification module 904 can be configured to identify a first noise characteristic associated with a shock channel signal. The second identification module 906 can be configured to identify a second noise characteristic associated with a non-shock ventricular channel signal, using the first noise characteristic. The third identification module 908 can be configured to identify a third noise characteristic, associated with an atrial channel signal, using the first and second noise characteristics. The noise classification module 910 can be configured to determine a noise classification using the first, second and third noise characteristics and using energy distribution in the time-frequency domain of at least one of the channels. In some examples, the intracardiac electrogram module 902 can be a part of a system including an implantable medical device placed within a patient's body. In other embodiments, the intracardiac electrogram module can be a portion of an implantable medical device including machine readable medium stored in memory and decipherable by a processor.

FIG.10 is a block diagram illustrating an example of an implantable cardiac function management system 1000 communicatively coupled to an external communication device. In this example, the system 1000 can include an implantable medical device (IMD) 1005, a physiological data sensor 1010, and an external communication device 1020. In an example, the IMD 1005 is a cardiac rhythm management (CRM) device used to provide cardiac rhythm therapy to a patient's heart. In an example, the physiological data sensor 1010 can be used to detect EGM data, such as including both sensed and evoked response depolarization information. In another example, the physiological data sensor 1010 can be used to monitor one or more other physiological parameters related to cardiac operations, such as heart rate, respiration rate, or blood pressure, among others. In some examples, multiple physiological data monitors can be employed to monitor multiple relevant physiological parameters.

The external communication device 1020 can be used for programming the IMD 1005, displaying data obtained from the IMD 1005, or for communicating data downloaded from the IMD 1005 to a physician or central monitoring station (not shown). In an example, the external device can include a personal computer, such as a laptop, configured to communicate with the IMD 1005. In an example, the external device communicates via a hardwired communication link with the IMD 1005. In an example, the external communication device 1020 communicates over a wireless communication link with the IMD 1005. In an example, the external communication device 1020 can receive data from the IMD 1005 and display that, such as on a computer display. In an example, the external communication device 1020 can also receive wireless communications initiated by the IMD 1005 for the purpose of downloading stored episode data for use by a physician in diagnosis or device programming. In this example, the external communication device 1020 can forward the data downloaded by the IMD 1005 to a central monitoring station over wired or wireless data connections. The wired data connections can include a digital subscriber line, cable modem, or a dial-up connection over a plain old telephone (POTS) line. This type of communication of data collected by an IMD 1005 is further explained in IMPLANTABLE MEDICAL DEVICE HAVING LONG-TERM WIRELESS CAPABILITIES, U.S. Patent 7,395,117 to Mazar et al.. This type of communication and IMD 1010 interaction with an external communication device is also further explained in METHOD AND APPARATUS FOR ENABLING DATA COMMUNICATION BETWEEN AN IMPLANTABLE MEDICAL DEVICE AND A PATIENT MANAGEMENT SYSTEM, U.S. Patent 7,127,300 to Mazar et al..

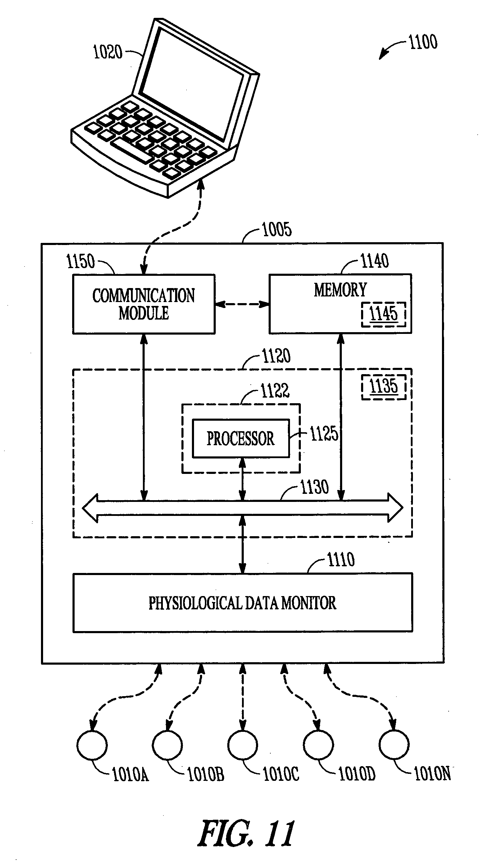

FIG.11 is a block diagram illustrating an example of an implantable cardiac function management system 1100 configured to enable adaptive data storage and download. In an example, a system 1100 can include an implantable medical device 1005, one or more physiological data sensors 1010A, 1010B, ..., 1010N (collectively hereinafter referred to as 1010), and an external communication device 1020. In an example of the system 1100, the IMD 1005 can include a physiological data monitor 1110, a processing module 1120, a memory 1140, and a communication module 1150. In some examples, the processing module 1120 can include a processor 1125 and a communication bus 1130. In certain examples, the processing module 1120 can also include a pathology detection circuit 1122. The pathology detection circuit includes the processor 1125 and is communicatively coupled to the physiological data monitor 1110. For example, signal sampling circuitry within the physiological data monitor 1110 can present digitized values of an electrical signal produced by the sensors 1010 to the pathology detection circuit 1122.

In some examples, the pathology detection circuit 1122 includes the processor 1125 and performs one or more detection algorithms that are embodied in instructions in software or firmware that are performable by the processor 1125. Such a processor may include a microprocessor, a digital signal processor (DSP), or application specific integrated circuit (ASIC).

In some examples, the processing module 1120 can also optionally include processor specific memory 1135 used to store data being manipulated by the processor 1125 or the pathology detection circuit 1122. In an example, the communication bus 1130 can enable communication between the physiological data monitor 1110, the processor 1125, the memory 1140, and the communication module 1150. The memory 1140 and the communication module 1150 can optionally communicate directly without routing through the communication bus 1130. In certain examples, the memory 1140 can include a main memory as well as one or more secondary memory circuits 1145. In an example, the secondary memory circuits 1145 can be used to temporarily store data while a connection to the external communication device 1020 is established.

In an example, the physiological data monitor 1110 can receive data from one or more physiological data sensors 1010. In certain examples, the physiological data sensors 1010 can include sensors implanted within the patient's body, also referred to as internal sensors. In other examples, the physiological data sensor(s) 1010 can include ambulatory or other external sensors such as worn or carried by the patient or adhered to a patient's skin or worn against a patient's skin. In some examples, the physiological data sensors 1010 can include both external and internal sensors. In an example, the physiological data sensors 1010 can include one or more of a heart sound sensor, a blood pressure sensor, a posture sensor, a respiratory sensor, an activity sensor, or a chemical sensor. In this example, the physiological data monitor 1110 can be configured to receive data from any or all of the sensors and to communicate the received data, such as to other portions of the IMD 1005 or to an external communication device 1020 via the communication module 1150.

In certain examples, the sensors 1010 can provide a time-varying electrical signal that is related to physiologic cardiovascular events of a subject. A non-exhaustive list of examples of such sensors 1010 include a cardiac signal sensing circuit, an intracardiac impedance sensing circuit, a transthoracic impedance sensing circuit, a blood pressure sensor, a blood gas sensor, a chemical sensor, a heart sound sensor, a posture sensor, and an activity sensor. In some examples, the IMD 1005 communicates with a sensor external to the IMD (not shown). The signals provided by this variety of sensors can be used to detect a pathological event or episode that a patient or subject is experiencing or has experienced.

For example, the IMD 1005 may be able to detect an arrhythmic event from a cardiac signal sensed using any of the electrodes described. The cardiac signal is representative of cardiac activity of a subject or patient. When a pathological episode such as an episode of arrhythmia is detected, the IMD 1005 may begin recording the cardiac signal (e.g., as an electrogram). The recorded cardiac signal, referred to generally as physiological data or monitored physiological data, may then be communicated to an external device. However, in general, every pathological episode detected by an IMD 1005 is stored in internal memory, such as memory 1140.

Returning to FIG.11, once received, the monitored physiological data can be transferred to the processor 1125 or stored directly in memory 1140. In this example, the memory 1140 can be accessed by the external communication device 1020 through the communication module 1150. In some examples, the communication module 1150 communicates to the external communication device 1020 over a communications link. As discussed above, the communications link between the external communication device 1020 and the IMD 1005 can be either wired or wireless.

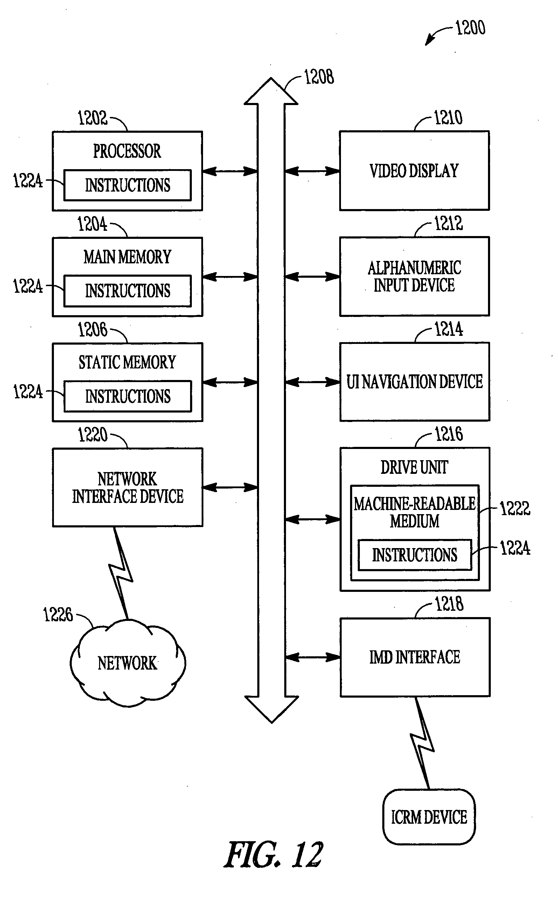

FIG.12 is a block diagram illustrating an example of an external communication and storage device. The system 1200 is a machine in the example form of a computer system 1200 within which instructions, for causing the machine to assist in the performance of any one or more of the methodologies discussed herein, may be executed. In certain examples, the machine operates as a standalone device or can be connected (e.g., networked) to other machines. In a networked deployment, the machine can operate in the capacity of a server or a client machine in a server-client network environment, or as a peer machine in a peer-to-peer (or distributed) network environment. The machine can be a personal computer (PC), a tablet PC, a set-top box (STB), a Personal Digital Assistant (PDA), a cellular telephone, a web appliance, a network router, switch or bridge, or any machine capable of executing instructions (sequential or otherwise) that specify actions to be taken by that machine. Further, while only a single machine is illustrated, the machine can include any collection of machines that individually or jointly execute a set (or multiple sets) of instructions to perform any one or more of the methodologies discussed herein.

The example computer system 1200 includes a processor 1202 (e.g., a central processing unit (CPU), a graphics processing unit (GPU) or both), a main memory 1204 and a static memory 1206, which communicate with each other via a bus 1208. The computer system 1200 can further include a video display unit 1210 (e.g., a liquid crystal display (LCD) or a cathode ray tube (CRT)). The computer system 1200 also includes an alphanumeric input device 1212 (e.g., a keyboard), a user interface (UI) navigation device 1214 (e.g., a mouse), a disk drive unit 1216, an implantable medical device interface 1218, and a network interface device 1220.
The implantable medical device interface can include a wired or wireless data connection with an implantable medical device. In certain examples, the system 1200 includes both a wired and a wireless data connection with an implantable medical device. In an example, the implantable medical device (IMD) interface allows information stored in the IMD to be downloaded to the computer system 1200 for storage and/or re-transmission to a treating physician or patient management system. In an example, the information downloaded from the IMD can be displayed on the video display unit 1210. In another example, the information downloaded can be processed by the processor 1202 prior to display on the video display unit 1210. In an example, the IMD interface can also upload information, including programming parameters for an implantable CRM device, back into the IMD.

The disk drive unit 1216 includes a machine-readable medium 1222 on which can be stored one or more sets of instructions and data structures (e.g., software) 1224 embodying or utilized by any one or more of the methodologies or functions described herein. The instructions 1224 can also reside, completely or at least partially, within the main memory 1204 or within the processor 1202 during execution thereof by the computer system 1200, the main memory 1204 and the processor 1202 also constituting machine-readable media.

While the machine-readable medium 1222 can be shown in an example embodiment to be a single medium, the term "machine-readable medium" can include a single medium or multiple media (e.g., a centralized or distributed database, or associated caches and servers) that store the one or more instructions or data structures. The term "machine-readable medium" can include any tangible medium that is capable of storing, encoding or carrying instructions for execution by the machine and that cause the machine to perform any one or more of the methodologies of the present application, or that is capable of storing, encoding or carrying data structures utilized by or associated with such instructions. The term "machine-readable medium" can include, but need not be limited to, solid-state memories, and optical and magnetic media. Specific examples of machine-readable media include non-volatile memory, including by way of example semiconductor memory devices, e.g., Erasable Programmable Read-Only Memory (EPROM), Electrically Erasable Programmable Read-Only Memory (EEPROM), and flash memory devices; magnetic disks including internal hard disks and removable disks; magneto-optical disks; and CD-ROM and DVD-ROM disks.

The instructions 1224 can further be transmitted or received over a communications network 1226 using a transmission medium. The instructions 1224 can be transmitted using the network interface device 1220 and any one of a number of transfer protocols (e.g., HTTP). Examples of communication networks include a local area network ("LAN"), a wide area network ("WAN"), the Internet, mobile telephone networks, Plain Old Telephone (POTS) networks, and wireless data networks (e.g., Wi-Fi and WiMax networks).

Certain examples are described herein as including logic or a number of components, modules, or mechanisms. A module is tangible unit capable of performing certain operations and may be configured or arranged in a certain manner. In examples, one or more computer systems (e.g., a standalone, client or server computer system) or one or more modules of a computer system (e.g., a processor or a group of processors) may be configured by software (e.g., an application or application portion) as a module that operates to perform certain operations as described herein.

In various examples, a module may be implemented mechanically or electronically. For example, a module may comprise dedicated circuitry or logic that is permanently configured (e.g., as a special-purpose processor) to perform certain operations. A module may also comprise programmable logic or circuitry (e.g., as encompassed within a general-purpose processor or other programmable processor) that is temporarily configured by software to perform certain operations. It will be appreciated that the decision to implement a module mechanically, in dedicated and permanently configured circuitry, or in temporarily configured circuitry (e.g., configured by software) may be driven by cost and time considerations.

Accordingly, the term "module" should be understood to encompass a tangible entity, be that an entity that is physically constructed, permanently configured (e.g., hardwired) or temporarily configured (e.g., programmed) to operate in a certain manner and/or to perform certain operations described herein. Considering examples in which modules are temporarily configured (e.g., programmed), each of the modules need not be configured or instantiated at any one instance in time. For example, where the modules comprise a general-purpose processor configured using software, the general-purpose processor may be configured as respective different modules at different times. Software may accordingly configure a processor, for example, to constitute a particular module at one instance of time and to constitute a different module at a different instance of time.

Modules can provide information to, and receive information from, other modules. Accordingly, the described modules may be regarded as being communicatively coupled. Where multiple of such modules exist contemporaneously, communications may be achieved through signal transmission (e.g., over appropriate circuits and buses) that connect the modules. In examples in which multiple modules are configured or instantiated at different times, communications between such modules may be achieved, for example, through the storage and retrieval of information in memory structures to which the multiple modules have access. For example, one module may perform an operation, and store the output of that operation in a memory device to which it is communicatively coupled. A further module may then, at a later time, access the memory device to retrieve and process the stored output. Modules may also initiate communications with input or output devices, and can operate on a resource (e.g., a collection of information).

### Additional Notes

The above detailed description includes references to the accompanying drawings, which form a part of the detailed description. The drawings show, by way of illustration, specific embodiments in which the invention can be practiced.

Method examples described herein can be machine or computer-implemented at least in part. Some examples can include a computer-readable medium or machine-readable medium encoded with instructions operable to configure an electronic device to perform methods as described in the above examples. An implementation of such methods can include code, such as microcode, assembly language code, a higher-level language code, or the like. Such code can include computer readable instructions for performing various methods. The code may form portions of computer program products. Further, the code may be tangibly stored on one or more volatile or non-volatile computer-readable media during execution or at other times. These computer-readable media may include, but are not limited to, hard disks, removable magnetic disks, removable optical disks (e.g., compact disks and digital video disks), magnetic cassettes, memory cards or sticks, random access memories (RAMs), read only memories (ROMs), and the like.

The invention is set out in the appended claims.

The present disclosure includes, inter alia, the following aspects:
1. A system comprising:
   a first identification circuit configured to identify a first noise characteristic associated with a sensing channel signal; and
   a noise classification circuit configured to determine a noise classification using the first noise characteristic and using information about energy distribution in a time-frequency domain of the sensing channel signal.
2. The system of aspect 1, further comprising a second identification circuit configured to identify a second noise characteristic, associated with a ventricular channel signal different from the sensing channel signal, using the first noise characteristic, wherein the noise classification circuit is configured to determine a noise classification using the first and second noise characteristics and using information about energy distribution in the time-frequency domain of at least one of the sensing channel signal or the ventricular channel signal.
3. The system of aspect 2, further comprising:
   a third identification circuit configured to identify a third noise characteristic, associated with a atrial channel signal, using the first and second noise characteristics; wherein the noise classification circuit is configured to determine noise classification using the first, second, and third noise characteristics and using information about the energy distribution in a time-frequency domain of at least one of the sensing channel signal, the ventricular channel signal, or the atrial channel signal.
4. The system of any one of aspects 1-3, further including a device programming circuit configured to provide a device programming or recommendation using the determined noise classification.
5. The system of any one of aspects 1-4, wherein the first identification circuit is configured to analyze the sensing channel signal for noise based on comparing a heart rate to an arrhythmia range.
6. The system of any one of aspects 1-5, wherein the first identification circuit is configured to analyze the sensing channel signal for noise by applying a filter to the sensing channel signal and determining the energy distribution above a specified frequency.
7. The system of aspect 2, wherein the second identification circuit is configured to analyze the ventricular channel signal for noise in a first interval when a heart rate is lower than or equal to a threshold to determine noise between candidate cardiac depolarizations and to analyze data in a second interval when the heart rate is higher than the threshold to determine noise between candidate cardiac depolarizations.
8. The system of aspect 7, wherein the second identification circuit is configured to analyze the ventricular channel signal for noise in the first interval including determining whether there is noise in the interval by comparing an amplitude of the ventricular channel signal to a first threshold.
9. The system of aspect 7, wherein the second identification circuit is configured to analyze the ventricular channel signal for noise in the second interval including applying a time-frequency transform to the ventricular channel signal, and determining a time instant having a maximal energy level and deriving a variability indicator of the time instant and comparing the variability indicator to a second threshold.
10. A method comprising:
   identifying a first noise characteristic associated with a sensing channel signal;
   using a circuit, determining a noise classification using the first characteristic and using information about an energy distribution in a time-frequency domain of the sensing channel signal.
11. The method of aspect 10, further comprising: identifying a second noise characteristic, associated with a ventricular channel signal different from the sensing channel signal, using the first noise characteristic, and determining a noise classification using the first and second noise characteristics and using information about energy distribution in the time-frequency domain of at least one of the sensing channel signal or the ventricular channel signal.
12. The method of aspect 11, further comprising identifying a third noise characteristic, associated with a atrial channel signal, using the first and second noise characteristics; wherein the noise classification circuit is configured to determine noise classification using the first, second, and third noise characteristics and using information about the energy distribution in a time-frequency domain of at least one of the sensing channel signal, the ventricular channel signal, or the atrial channel signal.
13. The method of aspect 10, further comprising providing a device programming or recommendation using the determined noise classification.
14. The method of aspect 11, further comprising analyzing the ventricular channel signal for noise in a first interval when a heart rate is lower than or equal to a threshold to determine noise between candidate cardiac depolarizations and analyzing data in a second interval when the heart rate is higher than the threshold to determine noise between candidate cardiac depolarizations.
15. The method of aspect 14, wherein analyzing the ventricular channel signal for noise in the first interval includes determining whether there is noise in the interval by comparing an amplitude of the ventricular channel signal to a first threshold.
16. The method of aspect 14, wherein analyzing the ventricular channel signal for noise in the second interval includes applying a time-frequency transform to the ventricular channel signal, and includes determining a time instant having a maximal energy level and deriving a variability characteristic of the time instant and comparing the variability characteristic to a second threshold.
17. An apparatus comprising:
   a first identification circuit configured for identifying a first noise characteristic associated with a shock channel signal;
   a second identification circuit configured for identifying a second noise characteristic associated with a ventricular rate sensing channel signal, using the first noise characteristic;
   a third identification circuit configured for identifying a third noise characteristic, associated with a atrial rate sensing channel signal, using the first and second noise characteristics; and
   a noise classification circuit configured for determining a noise classification using the first, second, and third noise characteristics using information about an energy distribution in a time-frequency domain of at least one of the shock channel signal, the ventricular rate sensing channel signal, or the atrial rate sensing channel signal.
18. The apparatus of aspect 17, wherein the second identification circuit is configured to analyze the ventricular channel signal for noise in a first interval when a heart rate is lower than or equal to a threshold to determine noise between cardiac depolarizations, and to analyze the ventricular channel signal for noise in a second interval when the heart rate is higher than the threshold to determine noise within a cardiac cycle.
19. The apparatus of aspect 18, wherein the second identification circuit is further configured to apply a time-frequency transform to the ventricular signal, and determine a time instant having a maximal energy level.
20. The apparatus of aspect 18, wherein the second identification circuit is configured to analyze the ventricular channel signal for noise in the second interval by determining whether a frequency distribution in the shock channel signal is within a narrow band and analyzing an energy distribution of the shock channel signal by applying a time frequency transform to the signal.

## Claims

1. A medical system (900) for classifying noise signals collected from an intracardiac electrogram and comprising:
a first identification circuit (904) configured to identify a first noise characteristic associated with a sensing channel signal;
a noise classification circuit (910) configured to determine a noise classification using the first noise characteristic and using information about energy distribution in a time-frequency domain of the sensing channel signal; and
a second identification circuit (906) configured to identify a second noise characteristic, associated with a ventricular channel signal different from the sensing channel signal, using the first noise characteristic, wherein the noise classification circuit (910) is configured to determine a noise classification using the first and second noise characteristics and using information about energy distribution in the time-frequency domain of at least one of the sensing channel signal or the ventricular channel signal.

2. The system of claim 1, further comprising:
a third identification circuit (908) configured to identify a third noise characteristic, associated with an atrial channel signal, using the first and second noise characteristics; wherein the noise classification circuit is configured to determine noise classification using the first, second, and third noise characteristics and using information about the energy distribution in a time-frequency domain of at least one of the sensing channel signal, the ventricular channel signal, or the atrial channel signal.

3. The system of claim 1, further including a device programming circuit configured to provide a device programming or recommendation using the determined noise classification.

4. The system of claim 1, wherein the first identification circuit (904) is configured to analyze the sensing channel signal for noise based on comparing a heart rate to an arrhythmia range.

5. The system of claim 1, wherein the first identification circuit (904) is configured to analyze the sensing channel signal for noise by applying a filter to the sensing channel signal and determining the energy distribution above a specified frequency.

6. The system of claim 1, wherein the second identification circuit (906) is configured to analyze the ventricular channel signal for noise in a first interval when a heart rate is lower than or equal to a threshold to determine noise between candidate cardiac depolarizations and to analyze data in a second interval when the heart rate is higher than the threshold to determine noise between candidate cardiac depolarizations.

7. The system of claim 6, wherein the second identification circuit (906) is configured to analyze the ventricular channel signal for noise in the first interval including determining whether there is noise in the interval by comparing an amplitude of the ventricular channel signal to a first threshold.

8. The system of claim 6, wherein the second identification circuit (906) is configured to analyze the ventricular channel signal for noise in the second interval including applying a time-frequency transform to the ventricular channel signal, and determining a time instant having a maximal energy level and deriving a variability indicator of the time instant and comparing the variability indicator to a second threshold.

9. A method for classifying noise signals collected from an intracardiac electrogram and comprising:
identifying a first noise characteristic associated with a sensing channel signal using a first identification circuit (904);
using a noise classification circuit (910), determining a noise classification using the first characteristic and using information about an energy distribution in a time-frequency domain of the sensing channel signal;
identifying a second noise characteristic, associated with a ventricular channel signal different from the sensing channel signal, using the first noise characteristic; and
determining a noise classification using the first and second noise characteristics and using information about energy distribution in the time-frequency domain of at least one of the sensing channel signal or the ventricular channel signal.

10. The method of claim 9, further comprising identifying a third noise characteristic, associated with an atrial channel signal, using the first and second noise characteristics; wherein the noise classification circuit (904) is configured to determine noise classification using the first, second, and third noise characteristics and using information about the energy distribution in a time-frequency domain of at least one of the sensing channel signal, the ventricular channel signal, or the atrial channel signal.

11. The method of claim 9, further comprising analyzing the ventricular channel signal for noise in a first interval when a heart rate is lower than or equal to a threshold to determine noise between candidate cardiac depolarizations and analyzing data in a second interval when the heart rate is higher than the threshold to determine noise between candidate cardiac depolarizations.

12. The method of claim 12, wherein analyzing the ventricular channel signal for noise in the first interval includes determining whether there is noise in the interval by comparing an amplitude of the ventricular channel signal to a first threshold.

13. The method of claim 12, wherein analyzing the ventricular channel signal for noise in the second interval includes:
applying a time-frequency transform to the ventricular channel signal;
determining a time instant having a maximal energy level;
deriving a variability characteristic of the time instant; and
comparing the variability characteristic to a second threshold.

## Patentansprüche

1. Medizinisches System (900) zum Klassifizieren von Störsignalen, die aus einem intrakardialen Elektrogramm gesammelt wurden, und aufweisend:
eine erste Identifizierungsschaltung (904), die zum Identifizieren einer mit einem Erfassungskanalsignal assoziierten, ersten Störungscharakteristik ausgebildet ist;
eine Störungsklassifizierungsschaltung (910), die zum Bestimmen einer Störungsklassifizierung unter Verwendung der ersten Störungscharakteristik und unter Verwendung von Informationen über eine Energieverteilung in einer Zeitfrequenzdomäne des Erfassungskanalsignals ausgestaltet ist; und
eine zweite Identifizierungsschaltung (906), die zum Identifizieren einer zweiten Störungscharakteristik, die mit einem ventrikulären Kanalsignal, das von dem Erfassungskanalsignal verschieden ist, assoziiert ist, unter Verwendung der ersten Störungscharakteristik ausgestaltet ist, wobei die Störungsklassifizierungsschaltung (910) ausgestaltet ist zum Bestimmen einer Störungsklassifizierung unter Verwendung der ersten und der zweiten Störungscharakteristik und unter Verwendung von Informationen über eine Energieverteilung in der Zeitfrequenzdomäne von zumindest einem von dem Erfassungskanalsignal oder dem ventrikulären Kanalsignal.

2. System nach Anspruch 1, weiterhin aufweisend:
eine dritte Identifizierungsschaltung (908), die zum Identifizieren einer dritten Störungscharakteristik, die mit einem Atrialkanalsignal assoziiert ist, unter Verwendung der ersten und der zweiten Störungscharakteristik ausgestaltet ist; wobei die Störungsklassifizierungsschaltung zur Bestimmung der Störungsklassifizierung unter Verwendung der ersten, zweiten und dritten Störungscharakteristik und unter Verwendung von Informationen über die Energieverteilung in einer Zeitfrequenzdomäne von zumindest einem von dem Erfassungskanalsignal, dem ventrikulären Kanalsignal und dem Atrialkanalsignal ausgestaltet ist.

3. System nach Anspruch 1, weiterhin enthaltend eine Vorrichtungsprogrammierungsschaltung, die ausgestaltet ist zum Vorsehen einer Vorrichtungsprogrammierung oder Empfehlung unter Verwendung der bestimmten Störungsklassifizierung.

4. System nach Anspruch 1, bei dem die erste Identifizierungsschaltung (904) zum Analysieren des Erfassungskanalsignals in Bezug auf Störungen auf der Grundlage eines Vergleichs einer Herzschlagfrequenz mit einem Arrhythmiebereich ausgestaltet ist.

5. System nach Anspruch 1, bei dem die erste Identifizierungsschaltung (904) zum Analysieren des Erfassungskanalsignals in Bezug auf Störungen durch Anwenden eines Filters für das Erfassungskanalsignal und Bestimmen der Energieverteilung oberhalb einer bestimmten Frequenz ausgestaltet ist.

6. System nach Anspruch 1, bei dem die zweite Identifizierungsschaltung (906) ausgestaltet ist zum Analysieren des ventrikulären Kanalsignals in Bezug auf Störungen in einem ersten Intervall, wenn eine Herzschlagfrequenz niedriger als ein oder gleich einem Schwellenwert ist, um Störungen zwischen Kandidatenherzdepolarisationen zu bestimmen, und zum Analysieren von Daten in einem zweiten Intervall, wenn die Herzschlagfrequenz höher als der Schwellenwert ist, um Störungen zwischen Kandidatenherzdepolarisationen zu bestimmen.

7. System nach Anspruch 6, bei dem die zweite Identifizierungsschaltung (906) ausgestaltet ist zum Analysieren des ventrikulären Kanalsignals in Bezug auf Störungen in dem ersten Intervall, enthaltend die Bestimmung, ob Störungen in dem Intervall auftreten, durch Vergleichen einer Amplitude des ventrikulären Kanalsignals mit einem ersten Schwellenwert.

8. System nach Anspruch 6, bei dem die zweite Identifizierungsschaltung (906) ausgestaltet ist zum Analysieren des ventrikulären Kanalsignals in Bezug auf Störungen in dem zweiten Intervall, enthaltend das Ausüben einer Zeit-Frequenz-Transformation bei dem ventrikulären Kanalsignal und das Bestimmen eines Zeitpunkts mit einem maximalen Energiepegel und Ableiten eines Variabilitätsindikators des Zeitpunkts und Vergleichen des Variabilitätsindikators mit einem zweiten Schwellenwert.

9. Verfahren zum Klassifizieren von Störungssignalen, die aus einem intrakardialen Elektrogramm gesammelt sind, und aufweisend:
Identifizieren einer ersten Störungscharakteristik, die mit einem Erfassungskanalsignal assoziiert ist, unter Verwendung einer ersten Identifizierungsschaltung (904);
Verwenden einer Störungsklassifizierungsschaltung (910), Bestimmen einer Störungsklassifizierung unter Verwendung der ersten Charakteristik und unter Verwendung von Informationen über eine Energieverteilung in einer Zeitfrequenzdomäne des Erfassungskanalsignals;
Identifizieren einer zweiten Störungscharakteristik, die mit einem ventrikulären Kanalsignal, das von dem Erfassungskanalsignal verschieden ist, assoziiert ist, unter Verwendung der ersten Störungscharakteristik; und
Bestimmen einer Störungsklassifizierung unter Verwendung der ersten und der zweiten Störungscharakteristik und unter Verwendung von Informationen über eine Energieverteilung in einer Zeitfrequenzdomäne von zumindest einem von dem Erfassungskanalsignal oder dem ventrikulären Kanalsignal.

10. Verfahren nach Anspruch 9, weiterhin aufweisend das Identifizieren einer dritten Störungscharakteristik, die mit einem Atrialkanalsignal assoziiert ist, unter Verwendung der ersten und der zweiten Störungscharakteristik; wobei die Störungsklassifizierungsschaltung (904) ausgestaltet ist zum Bestimmen einer Störungsklassifizierung unter Verwendung der ersten, zweiten und dritten Störungscharakteristik und unter Verwendung von Informationen über die Energieverteilung in einer Zeitfrequenzdomäne von zumindest einem von dem Erfassungskanalsignal, dem ventrikulären Kanalsignal oder dem Atrialkanalsignal.

11. Verfahren nach Anspruch 9, weiterhin aufweisend das Analysieren des ventrikulären Kanalsignals in Bezug auf Störungen in einem ersten Intervall, wenn eine Herzschlagfrequenz niedriger als ein oder gleich einem Schwellenwert ist, zum Bestimmen von Störungen zwischen Kandidatenherzdepolarisationen und das Analysieren von Daten in einem zweiten Intervall, wenn die Herzschlagfrequenz höher als der Schwellenwert ist, um Störungen zwischen Kandidatenherzdepolarisationen zu bestimmen.

12. Verfahren nach Anspruch 11, bei dem das Analysieren des ventrikulären Kanalsignals mit Bezug auf Störungen in dem ersten Intervall das Bestimmen enthält, ob Störungen in dem Intervall vorhanden sind, durch Vergleichen einer Amplitude des ventrikulären Kanalsignals mit einem ersten Schwellenwert.

13. Verfahren nach Anspruch 11, bei dem das Analysieren des ventrikulären Kanalsignals in Bezug auf Störungen in dem zweiten Intervall enthält:
Ausüben einer Zeit-Frequenz-Transformation bei dem ventrikulären Kanalsignal;
Bestimmen eines Zeitpunkts mit einem maximalen Energiepegel;
Ableiten einer Variabilitätscharakteristik des Zeitpunkts; und
Vergleichen der Variabilitätscharakteristik mit einem zweiten Schwellenwert.

## Revendications

1. Système médical (900) pour classifier des signaux de bruit collectés à partir d'un électrocardiogramme intracardiaque et comprenant :
un premier circuit d'identification (904) configuré pour identifier une première caractéristique de bruit associée à un signal de canal de détection ;
un circuit de classification de bruit (910) configuré pour déterminer une classification de bruit en utilisant la première caractéristique de bruit et en utilisant une information relative à la distribution d'énergie dans un domaine fréquence-temps du signal de canal de détection ; et
un second circuit d'identification (906) configuré pour identifier une seconde caractéristique de bruit, associée à un signal de canal ventriculaire différent du signal de canal de détection, en utilisant la première caractéristique de bruit, dans lequel le circuit de classification de bruit (910) est configuré pour déterminer une classification de bruit en utilisant les première et seconde caractéristiques de bruit et en utilisant une information relative à la distribution d'énergie dans le domaine fréquence-temps d'au moins un signal pris parmi le signal de canal de détection et le signal de canal ventriculaire.

2. Système selon la revendication 1, comprenant en outre :
un troisième circuit d'identification (908) configuré pour identifier une troisième caractéristique de bruit, associée à un signal de canal auriculaire, en utilisant les première et seconde caractéristiques de bruit ; dans lequel le circuit de classification de bruit est configuré pour déterminer une classification de bruit en utilisant les première, seconde et troisième caractéristiques de bruit et en utilisant une information relative à la distribution d'énergie dans le domaine fréquence-temps d'au moins un signal pris parmi le signal de canal de détection, le signal de canal ventriculaire et le signal de canal auriculaire.

3. Système selon la revendication 1, incluant en outre un circuit de programmation de dispositif configuré pour assurer une programmation ou recommandation de dispositif en utilisant la classification de bruit déterminée.

4. Système selon la revendication 1, dans lequel le premier circuit d'identification (904) est configuré pour analyser le signal de canal de détection quant à un bruit sur la base de la comparaison d'un rythme cardiaque avec une plage d'arythmie.

5. Système selon la revendication 1, dans lequel le premier circuit d'identification (904) est configuré pour analyser le signal de canal de détection quant à un bruit en appliquant un filtre au signal de canal de détection et en déterminant la distribution d'énergie au-delà d'une fréquence spécifiée.

6. Système selon la revendication 1, dans lequel le second circuit d'identification (906) est configuré pour analyser le signal de canal ventriculaire quant à un bruit dans un premier intervalle lorsqu'un rythme cardiaque est inférieur ou égal à un seuil afin de déterminer un bruit entre des dépolarisations cardiaques candidates et pour analyser des données dans un second intervalle lorsque le rythme cardiaque est supérieur au seuil afin de déterminer un bruit entre des dépolarisations cardiaques candidates.

7. Système selon la revendication 6, dans lequel le second circuit d'identification (906) est configuré pour analyser le signal de canal ventriculaire quant à un bruit dans le premier intervalle incluant la détermination de si oui ou non il y a un bruit dans l'intervalle en comparant une amplitude du signal de canal ventriculaire à un premier seuil.

8. Système selon la revendication 6, dans lequel le second circuit d'identification (906) est configuré pour analyser le signal de canal ventriculaire quant à un bruit dans le second intervalle incluant l'application d'une transformation fréquence-temps au signal de canal ventriculaire et la détermination d'un instant temporel présentant un niveau d'énergie maximal et la dérivation d'un indicateur de variabilité de l'instant temporel et la comparaison de l'indicateur de variabilité avec un second seuil.

9. Procédé pour classifier des signaux de bruit collectés à partir d'un électrocardiogramme intracardiaque et comprenant :
l'identification d'une première caractéristique de bruit associée à un signal de canal de détection en utilisant un premier circuit d'identification (904) ;
l'utilisation d'un circuit de classification de bruit (910), la détermination d'une classification de bruit en utilisant la première caractéristique et l'utilisation d'une information relative à une distribution d'énergie dans un domaine fréquence-temps du signal de canal de détection ;
l'identification d'une seconde caractéristique de bruit, associée à un signal de canal ventriculaire différent du signal de canal de détection, en utilisant la première caractéristique de bruit ; et
la détermination d'une classification de bruit en utilisant les première et seconde caractéristiques de bruit et en utilisant une information relative à la distribution d'énergie dans le domaine fréquence-temps d'au moins un signal pris parmi le signal de canal de détection et le signal de canal ventriculaire.

10. Procédé selon la revendication 9, comprenant en outre l'identification d'une troisième caractéristique de bruit, associée à un signal de canal auriculaire, en utilisant les première et seconde caractéristiques de bruit ; dans lequel le circuit de classification de bruit (904) est configuré pour déterminer une classification de bruit en utilisant les première, seconde et troisième caractéristiques de bruit et en utilisant une information relative à la distribution d'énergie dans le domaine fréquence-temps d'au moins un signal pris parmi le signal de canal de détection, le signal de canal ventriculaire et le signal de canal auriculaire.

11. Procédé selon la revendication 9, comprenant en outre l'analyse du signal de canal ventriculaire quant à un bruit dans un premier intervalle lorsqu'un rythme cardiaque est inférieur ou égal à un seuil afin de déterminer un bruit entre des dépolarisations cardiaques candidates et l'analyse des données dans un second intervalle lorsque le rythme cardiaque est supérieur au seuil afin de déterminer un bruit entre des dépolarisations cardiaques candidates.

12. Procédé selon la revendication 12, dans lequel l'analyse du signal de canal ventriculaire quant à un bruit dans le premier intervalle inclut la détermination de si oui ou non il y a un bruit dans l'intervalle en comparant une amplitude du signal de canal ventriculaire avec un premier seuil.

13. Procédé selon la revendication 12, dans lequel l'analyse du signal de canal ventriculaire quant à un bruit dans le second intervalle inclut :
l'application d'une transformation fréquence-temps au signal de canal ventriculaire ;
la détermination d'un instant temporel présentant un niveau d'énergie maximal ;
la dérivation d'une caractéristique de variabilité de l'instant temporel et la comparaison de la caractéristique de variabilité avec un second seuil.
